# EUROPÄISCHE PATENTSCHRIFT

(11) **EP 2 417 899 B1**
(45) Veröffentlichungstag und Bekanntmachung des Hinweises auf die Patenterteilung: **30.10.2013**
(21) Anmeldenummer: 11177169.7
(22) Anmeldetag: 10.08.2011
(51) Int. Cl.: A61B 1/008, A61B 1/005

(54) **Schaftelement für ein endoskopisches Instrument**
Shaft element for an endoscopic instrument
Elément d'arbre pour un instrument endoscopique

(30) Priorität: 13.08.2010 DE 102010034378
(43) Veröffentlichungstag der Anmeldung: 15.02.2012
(73) Patentinhaber: Karl Storz GmbH & Co. KG, 78532 Tuttlingen (DE)
(72) Erfinder: Bühs, Florian, 10777 Berlin (DE)
(74) Vertreter: Fugmann, Winfried

(56) Entgegenhaltungen:
- US-A- 4 790 624
- US-A- 6 036 636
- US-A1- 2003 023 142
- US-A1- 2007 135 803
- US-A1- 2008 027 285

## Beschreibung

Die vorliegende Erfindung betrifft ein Schaftelement für ein endoskopisches Instrument, insbesondere für ein Endoskop mit einer abwinkelbaren Endoskopspitze.

Endoskope werden heute für eine Vielzahl von Anwendungen in Medizin und Technik verwendet. Endoskope umfassen typischerweise einen starren oder flexiblen lang erstreckten Schaft, der zur Einführung in einen Hohlraum geeignet ist, und an dessen Spitze ein Endoskopobjektiv zur Erzeugung eines Bildes einer Szene in dem beobachteten Hohlraum angeordnet ist. Zur Aufnahme und Weiterleitung des endoskopischen Bildes vom distalen (d. h. beobachterfernen) Ende des Endoskops zum proximalen (beobachternahen) Ende können beispielsweise ein geordnetes Bündel von Lichtleitfasern innerhalb des Schafts oder ein elektronischer Bildaufnehmer, beispielsweise ein CCD-Chip, im Bereich des distalen Endes des Schafts sowie elektrische Leitungen innerhalb des Schafts vorgesehen sein. Bei starren Endoskopen kommen auch Linsensysteme, die eine Mehrzahl von Stablinsen umfassen können, zur Weiterleitung des Bildes zum Einsatz. Da in der Regel in dem beobachteten Hohlraum nicht ausreichend Licht vorhanden ist, ist ferner innerhalb des Schafts ein Lichtleitsystem vorgesehen, um ausreichend Licht an das distale Ende des Endoskops zu transportieren, wo es zur Beleuchtung des Hohlraums genutzt wird.

In einer Vielzahl von Anwendungen ist es wünschenswert, das distale Ende des Endoskopschafts, d. h. die Endoskopspitze, abwinkeln zu können, um nicht nur die Ausrichtung der Endoskopspitze, sondern auch die Blickrichtung einer in der Endoskopspitze angeordneten Optik verändern zu können. So kommen beispielsweise in der Gastroskopie, in der Sigmoidoskopie oder in der Bronchoskopie Endoskope zum Einsatz, bei denen der Schaft zumindest über einen großen Teil seiner Länge flexibel ausgebildet ist und die Endoskopspitze verstellt werden kann, um das Einführen des Endoskops in den Hohlraum zu erleichtern und die Blickrichtung den jeweiligen Anforderungen anzupassen. Aus ähnlichen Gründen kommen auch im industriellen Bereich Endoskope mit langem und flexiblem Schaft zum Einsatz, beispielsweise bei der Untersuchung größerer Maschinen mit komplexen Innenräumen, wie Turbinen oder Brennkammern.

Auch in der Laparoskopie kann eine abwinkelbare Endoskopspitze vorteilhaft sein. So kann der eingeschränkte Arbeitsraum, der durch das bei endoskopischen Operationen gasbefüllte Abdomen definiert ist, mit einem Endoskop, dessen Spitze abwinkelbar ist, komfortabler betrachtet werden. Hierfür ist insbesondere eine kurze und in einem großen Winkel abwinkelbare Endoskopspitze vorteilhaft. Diese kann beispielsweise die Spitze eines Endoskops mit ansonsten starrem Schaft bilden.

Zum Verstellen der abwinkelbaren Endoskopspitze kann diese, wie auch gegebenenfalls weitere Abschnitte des flexiblen Endoskopschafts, vom proximalen Ende des Endoskops her durch einen Bediener gesteuert werden.

Um eine steuerbare Abwinkelung eines Teils eines flexiblen Endoskopschafts zu ermöglichen, ist es bekannt, diesen mit einer Grundstruktur aus einzelnen gegeneinander schwenkbaren Segmenten auszubilden. So ist es beispielsweise aus DE 10 2007 001 580 A1 bekannt, dass aufeinander folgende Segmente durch jeweils ein einachsiges Schwenkgelenk miteinander verbunden sind und sich gegeneinander in einem kleinen Winkel verstellen lassen. Gemäß US 6,780,151 B2 sind zum Abwinkeln des Endoskopschafts entlang des Schafts eine Mehrzahl von Festkörpergelenken aufgereiht. Zum Verstellen des gesamten Schafts oder auch der Endoskopspitze sind Seilzüge vorgesehen, die durch das Endoskop bis zu einer im Handgriff befindlichen Betätigungseinrichtung geführt werden. Dabei kann jedes Segment gegenüber dem vorhergehenden bzw. nachfolgenden nur um einen kleinen Winkel geschwenkt werden. Eine Abwinkelung um einen erheblichen Winkel, beispielsweise 45° oder 90°, erfordert daher eine Vielzahl derartiger Segmente und somit eine relativ lange Endoskopspitze, wodurch die Verwendung in Anwendungen, bei denen ein kleiner Biegeradius notwendig ist, nicht möglich ist. Ferner beeinflussen sich bei der Abwinkelung die einzelnen Segmente gegenseitig. Ist eine Abwinkelung nur innerhalb einer Ebene möglich, so muss, um den gesamten Raum beobachten zu können, der Schaft des Endoskops um seine Längsachse gedreht werden; dabei dreht sich das mit der Kamera aufgenommene Bild jedoch entsprechend mit, was dem Bediener die Orientierung im Hohlraum erschwert. Wird andererseits durch alternierend um jeweils 90° verdrehte Schwenkachsen eine Abwinkelung in mehreren Ebenen ermöglicht, so ist hierfür die doppelte Anzahl an Segmenten erforderlich, was einen noch größeren minimalen Biegeradius zur Folge hat.

Aus US 2005/0131279 A1 ist ein Endoskopschaft bekannt, bei dem jedes Segment eine Verstellung um zwei aufeinander senkrecht stehende Achsen relativ zum nächsten bzw. vorhergehenden Segment erlaubt. Allerdings ist auch hierbei der Verstellwinkel relativ gering, so dass kein kleiner Biegeradius des Schafts bzw. der Spitze erzielt werden kann. Gemäß US 2008/0027285 A1 sind Schaftabschnitte eines Endoskops über mehrere durch Schraubenfedern gebildete flexible Verbindungsabschnitte miteinander verbunden, wobei die Verbindungsabschnitte mit Hilfe von Seilzügen in mehreren Ebenen gebogen werden können. Auf Grund der Flexibilität der Verbindungsabschnitte kann hierbei jedoch ein unerwünschter Versatz der Endoskopabschnitte gegeneinander auftreten. Ferner kommt es durch die Zugbelastung bei einer Abwinkelung zu einer Längenänderung, die die Bedienbarkeit erschwert und zu einem Verschleiß der Seilzüge führt.

In US 6,036,636 ist ein Endoskop mit einer abwinkelbaren Spitze offenbart, bei der die Spitze über eine Mehrzahl von Drähten mit dem Endoskopschaft verbunden ist, wobei durch eine Verschiebung der Drähte relativ zueinander eine Abwinkelung der Spitze erzielt werden kann. Das Endoskop beinhaltet wenigstens einen elastischen Draht, um den die Endoskopspitze abgewinkelt wird, und wenigstens einen elastischen Zug- bzw. Schubdraht, mit dem die Endoskopspitze abgewinkelt wird. Je nach Anzahl der Drähte kann die Spitze des Endoskops in alle Richtungen abgewinkelt werden. Hierbei besteht aufgrund der starken Biegung der Drähte und der erheblichen Belastung beim Abwinkeln die Gefahr einer Materialermüdung bzw. eines Materialbruchs, insbesondere bei einem großen Abwinkelungswinkel.

In US 2009/0171154 A1 ist ein Endoskop offenbart, dessen distales Ende abgeknickt wird, indem ein innerer Teil des Endoskops gegenüber einem äußeren Schaft verlängert wird, wobei der äußere Schaft und die abwinkelbare Spitze über eine Lasche miteinander verbunden sind. Hierdurch kann eine Abwinkelung nur in einer Ebene erzielt werden, sowie nur in einer Richtung.

Gemäß DE 299 07 430 U1 ist am distalen Ende eines starren Endoskopschafts eine Prismenkombination angeordnet, in der die optische Achse zweimal um jeweils 90° abgelenkt wird. Durch eine Drehmechanik können die Prismen um 360° gedreht werden, um eine beliebige Blickrichtung zu erzielen. Hierbei wird zur Verstellung jedoch eine aufwändige Mechanik benötigt, außerdem erhöht sich der gesamte Außendurchmesser des Schafts, so dass die Anwendungsmöglichkeiten eingeschränkt sind.

Es ist die Aufgabe der vorliegenden Erfindung, ein Schaftelement für ein endoskopisches Instrument anzugeben, das die oben genannten Nachteile nicht aufweist und das insbesondere eine Abwinkelung in alle Raumrichtungen ermöglicht.

Ein endoskopisches Instrument der eingangs genannten Art weist einen Schaft auf, der insbesondere lang erstreckt und zur Einführung in einen Hohlraum geeignet ist. Der Schaft kann starr oder flexibel ausgebildet sein. Im Rahmen der vorliegenden Anmeldung wird unter endoskopisches Instrument eines verstanden, das zur Einführung in einen Hohlraum und zur Beobachtung innerhalb des Hohlraums und/oder zur Durchführung von Manipulationen innerhalb des Hohlraums geeignet ist, wobei nicht unbedingt die Möglichkeit der optischen Beobachtung vorhanden sein muss. Endoskope zur Einführung in einen Hohlraum und zur Beobachtung innerhalb des Hohlraums sind Teil der endoskopischen Instrumente im Sinne dieser vorliegenden Anmeldung.

Ein Abschnitt des Schafts eines derartigen endoskopischen Instruments weist erfindungsgemäß eine Mehrzahl von Segmenten auf, von denen zumindest zwei gegeneinander verkippt werden können. Hierdurch kann beispielsweise eine abwinkelbare Spitze, die mit einem distalen der beiden gegeneinander verkippbaren Segmente verbunden ist, gegenüber dem übrigen Schaft, der mit dem proximalen der beiden Segmente verbunden ist, abgewinkelt werden. Ebenso kann ein distaler Abschnitt des Schafts, der mit einem distalen der beiden Segmente verbunden ist, gegenüber einem proximalen Abschnitt des Schafts, der mit dem proximalen der beiden Segmente verbunden ist, abgewinkelt werden. Ein Schaft umfasst somit mindestens zwei Segmente, die gegeneinander abgewinkelt werden können und dadurch ein Schaftelement zur Abwinkelung einer Spitze oder eines Abschnitts eines Schafts bilden.

Erfindungsgemäß umfasst ein Schaftelement für ein endoskopisches Instrument ein erstes, proximalseitig angeordnetes Schaftsegment und ein zweites, distalseitig angeordnetes Schaftsegment, die miteinander über drei Verbindungselemente verbunden sind. Die Verbindungselemente weisen jeweils ein erstes zweiachsiges Gelenk auf, das Kippbewegungen um zwei zueinander senkrechte Achsen, die jeweils quer zu einer Längsachse des Schafts, insbesondere einer Längsachse des zweiten Schaftsegments, stehen, ermöglicht. Die ersten Gelenke sind insbesondere derart ausgebildet, dass sie ein Kippen in alle Richtungen zulassen, jedoch keine Rotation um eine Längsachse des Verbindungselements bzw. des zweiten Schaftsegments. Die drei ersten Gelenke sind in Form eines Dreiecks angeordnet, insbesondere in Form eines quer zur Längsachse des zweiten Schaftsegments stehenden Dreiecks. Die drei ersten Gelenke liegen somit nicht auf einer geraden Linie, so dass hierdurch eine eindeutige Lage des zweiten Schaftsegments relativ zum ersten Schaftsegment bestimmt ist. Insbesondere liegen die drei ersten Gelenke nicht in einer Längsebene des zweiten Schaftsegments bzw. in einer gestreckten Stellung des Schaftelements nicht in einer Längsebene des Schafts.

Erfindungsgemäß ist weiterhin ein erstes der drei Verbindungselemente derart mit dem ersten Schaftsegment verbunden, dass das erste Verbindungselement in einer Längsrichtung, insbesondere in einer Längsrichtung des ersten Schaftsegments, gegenüber dem ersten Schaftsegment nicht verschiebbar ist. Insbesondere kann das erste Verbindungselement fest bzw. starr mit dem ersten Schaftsegment verbunden sein. Ein zweites und ein drittes der drei Verbindungselemente sind gegenüber dem ersten Schaftsegment in einer Längsrichtung des Schafts, insbesondere in der Längsrichtung des ersten Schaftsegments, unabhängig voneinander verschiebbar. Durch Verschiebung des zweiten und des dritten Verbindungselements relativ zum ersten Schaftsegment sowie gegebenenfalls des zweiten und des dritten Verbindungselements gegeneinander wird eine Verkippung des zweiten Schaftsegments relativ zum ersten Schaftsegment in alle Raumrichtungen ermöglicht. Die übrigen Abschnitte des Schafts, die mit dem ersten bzw. dem zweiten Schaftsegment verbunden sind, können sowohl starr als auch flexibel ausgebildet sein.

Dadurch, dass das zweite Schaftsegment über drei Verbindungselemente mit dem ersten Schaftsegment verbunden ist, die jeweils ein erstes zweiachsiges Gelenk aufweisen, und dass ein erstes der drei Verbindungselemente zumindest bezüglich einer Längsrichtung fest mit dem ersten Schaftsegment verbunden und ein zweites und ein drittes der drei Verbindungselemente gegenüber dem ersten Schaftsegment in einer Längsrichtung des Schafts unabhängig voneinander verschiebbar sind, wird auf einfache Weise eine Abwinkelung des zweiten Schaftsegments relativ zum ersten in alle Raumrichtungen ermöglicht, ohne dass die Verbindungselement durch starke Biegungen belastet würden, die zu einer Ermüdung oder zu einem Bruch des Materials führen könnten. Dadurch kann auch eine kurze Spitze in alle Raumrichtungen abgewinkelt werden. Ferner ist keine Rotation des Schafts notwendig, um alle Blickrichtungen zu realisieren. Das aufgenommene endoskopische Bild bleibt daher aufrecht stehen.

Gemäß einer bevorzugten Ausführungsform der Erfindung sind die ersten Gelenke in Form eines gleichseitigen Dreiecks angeordnet, das quer zur Längsrichtung des Schafts bzw. quer zu einer Längsachse des zweiten Schaftsegments steht. Hierdurch wird eine Steuerung der Abwinkelung in allen Raumrichtungen unter einem nahezu konstanten Übersetzungsverhältnis zwischen der Verschiebung des zweiten und/oder dritten Verbindungselements und dem erzielten Kippwinkel erreicht, wodurch die Bedienung vereinfacht wird.

In weiter vorteilhafter Weise sind die ersten Gelenke in oder nahe einer proximalen Endebene des zweiten Schaftsegments angeordnet. Dadurch wird eine raumsparende und stabile Anordnung erzielt. Insbesondere kann eine abwinkelbare Spitze des endoskopischen Instruments besonders kurz ausgebildet sein, was einen Einsatz in engen oder flachen Hohlräumen ermöglicht.

Gemäß einer besonders bevorzugten Ausführungsform weisen das zweite und das dritte Verbindungselement jeweils ein zweites zweiachsiges Gelenk auf. Dieses zweite Gelenk kann insbesondere in ähnlicher Weise wie das erste Gelenk ausgeführt sein. Dadurch, dass das zweite und das dritte Verbindungselement jeweils ein zweites Gelenk aufweisen, wird ein besonders verschleißfester Aufbau erreicht, da das zweite und das dritte Verbindungselement nicht auf Biegung beansprucht werden und deshalb im Bereich des Schaftelements beispielsweise mit starren Stäben ausgeführt werden können. Auch die zweiten Gelenke sind insbesondere rotationsstarr ausgebildet.

In einer weiteren bevorzugten Ausführungsform der Erfindung sind das zweite und das dritte Verbindungselement jeweils als Schubstange ausgebildet, die zumindest abschnittsweise starr ist. Insbesondere kann die Schubstange über ihre gesamte Länge starr sein, so dass eine Abwinkelung der Schubstange nur in den zweiten Gelenken erfolgt. Die Schubstange kann aber auch teilweise flexibel ausgebildet sein, um in einem flexiblen Schaft der Verformung des Schafts folgen zu können. Die Schubstange ist insbesondere zur Übertragung von Schub- und Zugbewegungen ausgebildet und wird daher auch als Zugstange bezeichnet. Dadurch, dass die Verbindungselemente jeweils zumindest abschnittsweise starr ausgebildet sind, wird eine besonders verschleißfreie Ausführung ermöglicht.

Weiterhin ist es bevorzugt, dass das zweite und das dritte Verbindungselement bzw. die Schubstangen, die das zweite und das dritte Verbindungselement bilden, auf einen Querschnitt des ersten Segments bezogen, in dessen Randbereich angeordnet sind. Hierdurch wird einerseits ein besonders großer Hebelarm für die Abwinkelung des zweiten Segments geschaffen, andererseits verbleibt innerhalb des ersten Segments ein besonders großer Freiraum zur Durchführung von Kabeln, Lichtleitern oder anderen Kanälen, die vom proximalen zum distalen Ende des endoskopischen Instruments oder umgekehrt geführt werden müssen.

Gemäß einer weiteren Ausführungsform setzt das erste Verbindungselement in einem Randbereich des zweiten Segments an, insbesondere in einem Randbereich einer proximalen Endebene des zweiten Segments. Dies hat den Vorteil eines besonders großen Hebelarms für die Abwinkelung und eines großen verbleibenden Freiraums im inneren Bereich des zweiten Segments, insbesondere dann, wenn das zweite und das dritte Verbindungselement ebenfalls in einem Randbereich, beispielsweise im gegenüberliegenden Randbereich, ansetzen.

Gemäß einer anderen Ausführungsform setzt das erste Verbindungselement mittig, insbesondere in der Mitte einer proximalen Endfläche, am zweiten Segment an. Hierdurch wird erreicht, dass eine Abwinkelung des zweiten Segments gegenüber dem ersten Segment in symmetrischer Weise in allen Richtungen möglich ist.

Hierbei kann in besonders vorteilhafter Weise das erste Verbindungselement im Randbereich des ersten Segments, insbesondere in dem Randbereich, in dem das zweite und das dritte Verbindungselement bzw. die entsprechenden Schubstangen durch das erste Segment hindurch geführt sind, an diesem ansetzen. Auch hierdurch kann ein großer Raum zur Durchführung von Leitungen durch das erste Segment erzielt werden.

Das erste Verbindungselement kann beispielsweise die Form eines abgekröpften Auslegers haben, der die Position des ersten Gelenkes des ersten Verbindungselements gegenüber dem ersten Segment festlegt. Bei dieser Ausführungsform wird der Bauraum innerhalb des ersten Segments in geringstmöglicher Weise durch die Verbindungselemente eingeschränkt.

Die zweiachsigen Gelenke können insbesondere als Kardangelenke ausgebildet sein mit zwei aufeinander senkrecht stehenden Achsen, die in einer gestreckten Stellung des Schaftelements senkrecht zur Längsrichtung des Schafts ausgerichtet sind. Ein derartiges Kardangelenk hat den Vorteil eines einfachen Aufbaus und großer Steifheit gegenüber Drehbewegungen um die Längsachse.

Die Gelenke können aber auch durch eine Schraubenfeder, ein Paket von Tellerfedern, ein Elastomergelenk oder eine Kombination von Festkörpergelenken gebildet sein. Ferner können die Gelenke mit Hilfe eines Faltenbalgs, der aus Metall oder aus Kunststoff hergestellt sein kann, realisiert werden. Diese Lösungen haben den Vorteil eines einfachen und miniaturisierbaren Aufbaus.

Der Antrieb der Schubstangen kann durch manuelle Betätigung vom proximalen Ende des endoskopischen Instruments in an sich bekannter Weise erfolgen. Gemäß einer bevorzugten Ausführungsform erfolgt der Antrieb über Aktuatoren, die innerhalb des ersten Segments oder innerhalb Schafts angeordnet sind. Derartige Aktuatoren können beispielsweise elektromotorisch, gegebenenfalls mit Linearschneckenaufsatz, hydraulisch oder auch pneumatisch realisiert sein. Auch Linearantriebe, künstliche Muskeln oder Piezolinearantriebe sind möglich. Die gleichen Antriebsformen können aber auch am proximalen Ende, beispielsweise innerhalb eines Handgriffs, realisiert sein, wobei die Übertragung über Schubstangen zum Schaftelement erfolgen kann.

Die Ansteuerung der Abwinkelungsbewegung kann insbesondere mit zwei einfachen Motoren erfolgen. Durch Erfassen einer Motorposition ist ein eindeutiger Rückschluss auf die Stellung des zweiten Segments und damit ggf. auf die Position der Spitze möglich. Diese Information kann bei Anwendungen mit Virtual Reality, Augmented Reality oder integrierter Navigation dazu genutzt werden, um beispielsweise den Abknickwinkel in einem endoskopischen Bild anzuzeigen. Ferner kann hierdurch eine Bewegungssteuerung der Spitze des als Endoskop ausgebildeten endoskopischen Instruments beispielsweise durch Sprachsteuerung oder Eye-Tracking vereinfacht werden.

Zum Schutz der Leitungen und/oder der Abwinkelungsmechanik gegen Verschmutzung, Beschädigungen und gegen das Eindringen von Dampf beim Autoklavieren kann eine Umhüllung vorgesehen sein, beispielsweise ein Faltenbalg aus Metall oder aus einem Hochleistungskunststoff wie PTFE. Die beweglichen Bauteile bzw. das Schaftelement können auch mit einer Schutzschicht oder einer Elastomerhülle umgeben werden.

Ein erfindungsgemäßes endoskopisches Instrument umfasst einen Schaft mit einer abwinkelbaren Spitze sowie einen proximalen Bedienteil mit Bedienelementen und elektrischen Anschlüssen, Lichtanschlüssen und/oder weiteren Anschlüssen. Der Schaft umfasst mindestens ein erfindungsgemäßes Schaftelement; insbesondere kann eine Spitze über das Schaftelement mit dem übrigen Bereich des Schafts, der starr oder flexibel ausgebildet sein kann, abwinkelbar verbunden sein. In bevorzugter Weise sind innerhalb der Endoskopspitze ein Endoskopobjektiv und ein elektronischer Bildaufnehmer, beispielsweise ein CCD-Chip, angeordnet ("chip on the tip") sowie ggf. die Lichtausgänge einer Beleuchtungsoptik. Die Versorgungs- und Datenleitungen des elektronischen Bildaufnehmers sowie die Lichtleiter der Beleuchtungsoptik sind durch den Schaft bis zum proximalen Ende geführt. Die Endoskopspitze kann aber auch eine Lichtquelle umfassen, etwa eine lichtemittierende Diode (LED), deren Versorgungs- und ggf. Kühlleitungen durch den Schaft verlaufen. Ebenso können Kanäle zum Durchführen endoskopischer Instrumente und/oder Spül- bzw. Saugkanäle durch den Schaft geführt werden.

In vorteilhafter Weise können in einem endoskopischen Instrument mehrere erfindungsgemäße Schaftelemente hintereinander angeordnet sein. Dabei stellt jeweils das zweite Schaftsegment eines ersten Schaftelements das erste Segment eines zweiten Schaftelements dar bzw. ist mit diesem verbunden. Hierdurch werden ein größerer Abwinkelungsbetrag und/oder eine mehrfache Abwinkelung des Schafts ermöglicht. Dabei können jeweils zwei Aktuatoren pro abwinkelbarem Segment vorgesehen sein, die beispielsweise in den jeweiligen ersten Segmenten im Schaft angeordnet sein können. Hierdurch bauen die einzelnen Abwinkelungen aufeinander auf, ohne sich gegenseitig zu beeinflussen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 eine vereinfachte Darstellung des Aufbaus eines Ausführungsbeispiels der Erfindung;
Fig. 2 das Ausführungsbeispiel gemäß Fig. 1 in einer ersten Abwinkelungsstellung;
Fig. 3 das Ausführungsbeispiel der Fig. 1 in einer zweiten Abwinkelungsstellung;
Fig. 4a und 4b ein zweites Ausführungsbeispiel der Erfindung in schematischer Darstellung in gestreckter bzw. in abgewinkelter Stellung;
Fig. 5a und 5b ein drittes Ausführungsbeispiel der Erfindung in schematischer Darstellung in gestreckter bzw. in abgewinkelter Stellung;
Fig. 6a bis 6c verschiedene Ausführungsformen eines zweiachsigen Gelenks zur Verwendung in einem erfindungsgemäßen Schaftelement;
Fig. 7a bis 7d weitere Ausführungsbeispiele eines zweiachsigen Gelenks zur Verwendung bei einem erfindungsgemäßen Schaftelement.

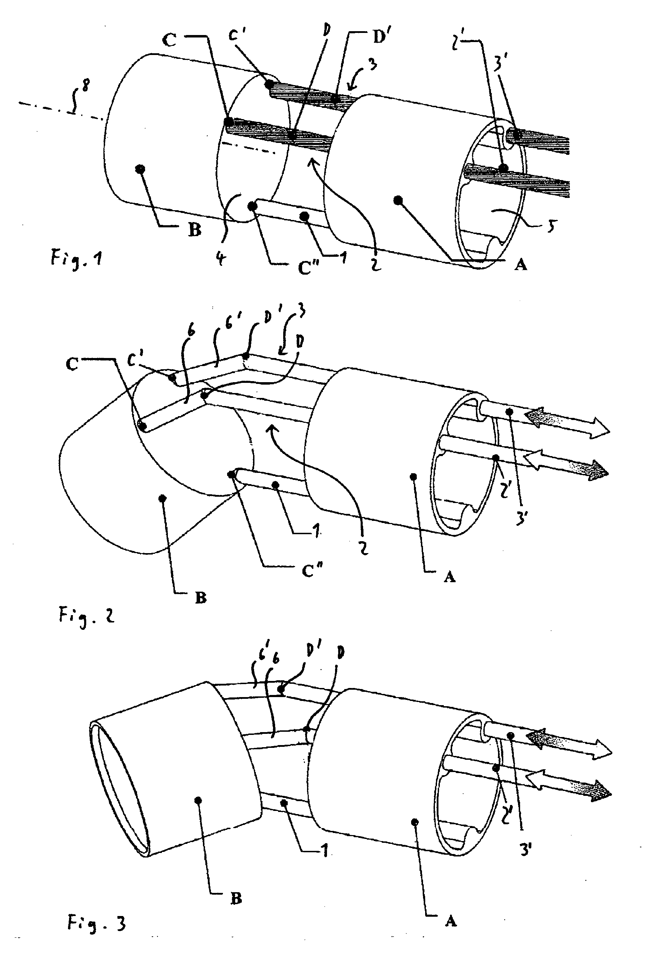

Wie in Fig. 1 gezeigt, weist ein Schaftelement gemäß einem ersten Ausführungsbeispiel der Erfindung zwei Segmente A, B auf, nämlich ein proximales Segment A und ein distales Segment B, welches gegenüber dem ersten Segment A verstellt, d. h. abgewinkelt werden kann. Zwischen den beiden Segmenten befinden sich drei Verbindungselemente 1, 2, 3. Die Verbindungselemente 1, 2, 3 weisen jeweils ein Gelenk C, C', C" an der Verbindungsstelle zum Segment B auf. Diese Gelenke sind derart ausgeführt, dass sie ein Kippen in alle Richtungen zulassen, jedoch keine Rotation um eine zur Längsachse 8 des zweiten Segments B parallele Achse bzw. um die Längsachse des Schafts, die in der in Fig. 1 gezeigten gestreckten Stellung mit der Längsachse 8 des zweiten Segments B zusammenfällt. Ein erstes Verbindungselement 1 ist im Segment A fixiert.

Zwei weitere Verbindungselemente 2, 3 sind als Schubstangen 2', 3' ausgeführt bzw. jeweils mit einer Schubstange 2', 3' verbunden, wobei die Schubstangen 2', 3' innerhalb des Segments A verschiebbar gelagert und zur Betätigung nach proximal durch das erste Segment A durchgeführt sind. Die beiden Verbindungselemente 2, 3 weisen jeweils ein weiteres zweiachsiges Gelenk D, D' auf. Auch diese Gelenke erlauben ein Kippen in alle Richtungen, jedoch keine Rotation um eine zur Achse der jeweiligen Schubstange parallele Achse. Wie in Fig. 1 gezeigt, sind die beiden Schubstangen 2', 3' im Randbereich des Segments A geführt, um möglichst viel Raum für innerhalb des Segments A geführte Leitungen, beispielsweise elektrische Versorgungs- und Datenleitungen oder Lichtleiter, zu lassen. Gemäß dem in Fig. 1 dargestellten Ausführungsbeispiel ist das erste Verbindungselement 1 ebenfalls im Randbereich der Segmente A, B angeordnet.

Die Anlenkpunkte der Verbindungselemente 1, 2, 3 am zweiten Segment B bzw. die Gelenke C, C', C" bilden näherungsweise ein gleichseitiges Dreieck, dessen Fläche senkrecht auf der Längsachse 8 des zweiten Segments B steht und eine proximale Endebene 4 des zweiten Segments B definiert. Das erste Segment A weist ein Lumen 5 zur Durchführung der in Fig. 1 nicht dargestellten elektrischen Leitungen, Lichtleitungen und/oder weiteren Kanäle auf. Auch das zweite Segment B weist einen derartigen Hohlraum auf (nicht dargestellt).

Gemäß Fig. 2 erfolgt die Abwinkelung von Segment B gegenüber Segment A durch eine lineare Verschiebung einer oder beider Schubstangen 2', 3' in Richtung der Pfeile. Durch Verlängerung oder Verkürzung der Verbindungselemente 2, 3 bzw. des zwischen den beiden Segmenten befindlichen Teils der Schubstangen 2', 3' werden die Gelenke C, C', C", D, D' in definierter Weise abgeknickt und hierdurch eine Schrägstellung des Segments B relativ zum Segment A erreicht. Dabei können die Verbindungselemente 2, 3 in ihrem distalen, jenseits des Gelenks D, D' angeordneten Abschnitt jeweils als starrer Stab 6, 6' ausgebildet sein. Durch die Verschiebung der Schubstangen 2', 3' sind die Richtung und die Größe der Abwinkelung des Segments B bestimmt. Wird die Verschiebung der Schubstangen 2', 3' gemessen, so kann auf die tatsächliche Abwinkelung und damit auf die Blickrichtung einer mit Segment B verbundenen Spitze geschlossen werden. Dies kann beispielsweise für Virtual-Reality-Anwendungen genutzt werden.

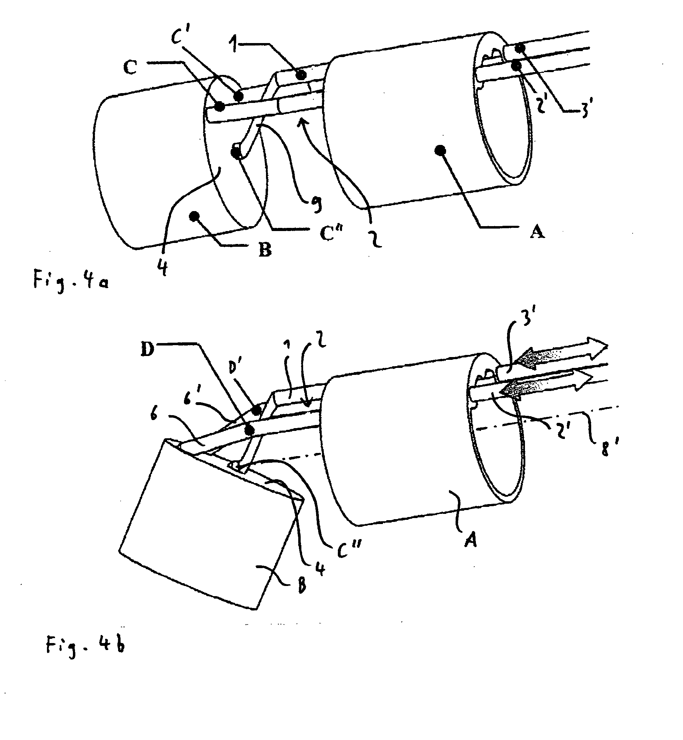

Gemäß Fig. 1 erfolgt die Abwinkelungsbewegung des zweiten Segments immer um das Gelenk C", also nicht symmetrisch zur Mittellängsachse 8' des ersten Segments. Eine symmetrische Knickbewegung lässt sich durch eine Modifikation gemäß Fig. 4a und 4b erreichen. Dabei ist das feststehende Verbindungselement 1 als doppelt abgeknickter bzw. gekröpfter Ausleger 9 des ersten Segments A ausgebildet, der im Bereich zwischen den Schubstangen 2', 3' am ersten Segment A ansetzt. Der Ansatzpunkt des ersten Verbindungselements 1 am zweiten Segment B, d. h. das Gelenk C", ist demgegenüber mittig in der proximalen Endebene 4 des zweiten Segments B angeordnet.

Fig. 4b zeigt eine abgewinkelte Stellung des Ausführungsbeispiels aus Fig. 4a. Wie in Fig. 4b zu erkennen ist, kann durch eine Verschiebung der Schubstangen 2', 3' in Richtung der Pfeile eine Abwinkelung des zweiten Segments B relativ zum ersten Segment A um einen Drehpunkt, der auf einer Mittellängsachse 8' des ersten Segments A liegt, gesteuert werden.

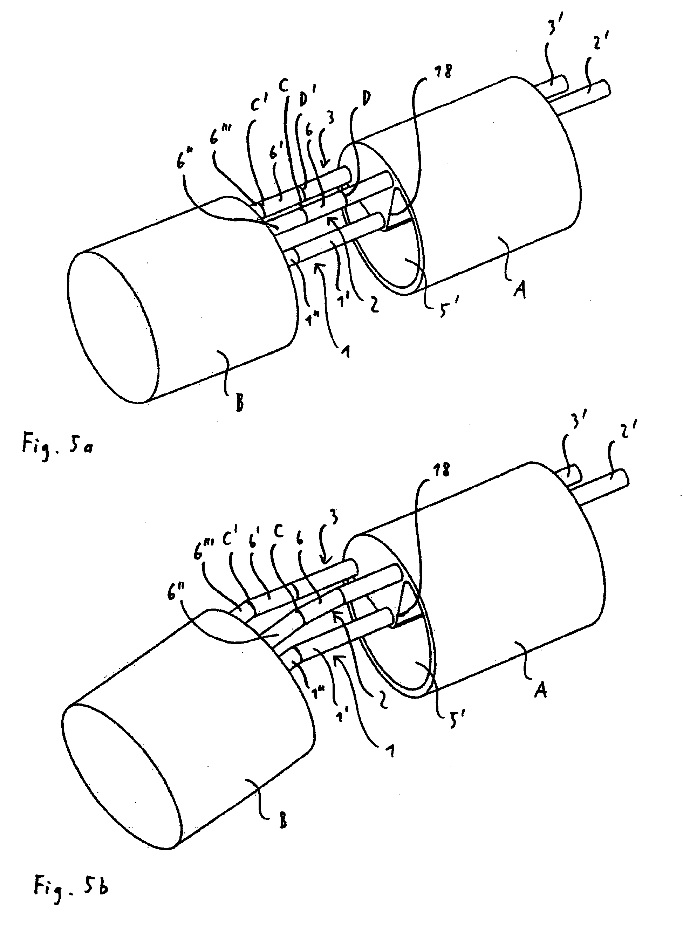

Ein weiteres Ausführungsbeispiel der Erfindung ist in Fig. 5a und 5b schematisch dargestellt. Die Gelenke C", C, C' der Verbindungselemente 1, 2, 3 bilden bei dieser Ausführungsform ein Dreieck, das jedoch gegenüber einer proximalen Endebene des zweiten Schaftelements B nach proximal versetzt ist. Hierfür ist das erste Verbindungselement 1 als abschnittsweise starrer Stab ausgebildet, wobei ein proximaler Abschnitt 1' starr mit dem ersten Schaftsegment A und ein distaler Abschnitt 1" starr mit dem zweiten Schaftsegment B verbunden ist. Der proximale Abschnitt 1' ist mit dem distalen Abschnitt 1" über das Gelenk C" verbunden. Das zweite und das dritte Verbindungselement 2, 3 sind nicht nur jeweils in ihrem distal des weiteren Gelenks D, D' angeordneten Abschnitt als starrer Stab 6, 6' ausgebildet, sondern weisen einen als starrer Stab 6", 6''' ausgebildeten weiteren distalen Abschnitt auf, der über das Gelenk C, C' mit dem Stab 6, 6' verbunden ist. Die letztgenannten distalen Abschnitte des zweiten und des dritten Verbindungselements 2, 3 sind jeweils starr mit dem zweiten Schaftsegment B verbunden. Insbesondere können das zweite und das dritte Verbindungselement jeweils als gelenkiger Stab mit zwei zweiachsigen Gelenken ausgebildet sein.

Fig. 5b zeigt eine abgewinkelte Stellung des Ausführungsbeispiels aus Fig. 5a. Wie in Fig. 5b symbolisch durch eine Verkürzung der aus dem proximalen Schaftelement A nach proximal herausragenden Abschnitte der Schubstangen 2', 3' angedeutet ist, kann durch eine Verschiebung der Schubstangen 2', 3' eine Abwinkelung des distalen Schaftsegments B um einen Drehpunkt, der durch das Gelenk C" des ersten Verbindungselements 1 bestimmt ist, erreicht werden.

Wie in Fig. 5a und 5b weiterhin zu erkennen ist, kann das erste Verbindungselement an einem mit einem Mantel des ersten Schaftsegments A innenseitig verbundenen Vorsprung 18 ansetzen. Hierdurch verbleibt trotz der Einschränkung durch das nahezu mittig ansetzende erste Verbindungselement 1 ein relativ großes Lumen 5' zur Aufnahme weiterer Funktionselemente des endoskopischen Instruments.

In einem endoskopischen Instrument können mehrere der beschriebenen Schaftelemente hintereinander angeordnet sein. Dabei kann beispielsweise die proximale Endfläche eines ersten Segments A entsprechend der proximalen Endfläche 4 des zweiten Segments B ausgebildet sein, woran Verbindungselemente zu einem weiteren proximalen Segment ansetzen (nicht dargestellt). Durch die Hintereinanderanordnung mehrerer Segmente der beschriebenen Art bzw. mehrerer Schaftelemente kann ein besonders flexibler Aufbau eines Schafts erreicht werden.

Anstelle von durch das proximale Segment A hindurch geführten Schubstangen 2', 3' können die Verbindungselemente 2, 3 auch durch Aktuatoren, die innerhalb des proximalen Segments A angeordnet sind, betätigt werden. Diese Aktuatoren werden vom proximalen Ende des endoskopischen Instruments über Bedienelemente und innerhalb des Schafts angeordnete Steuerleitungen gesteuert. Auch in diesem Fall ist eine genaue Positionierung der einzelnen Elemente möglich.

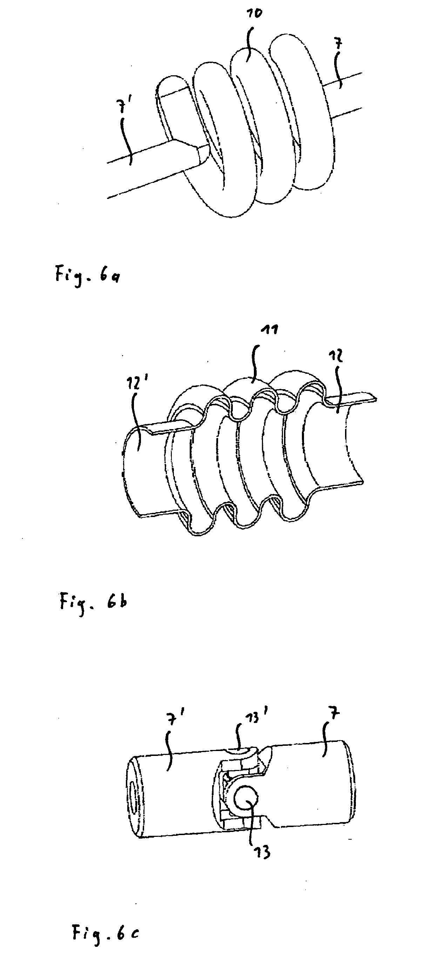

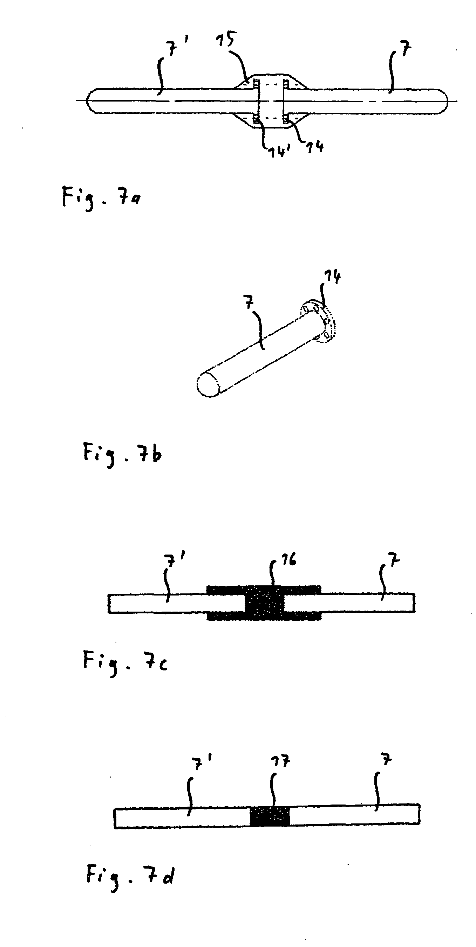

Die bei einem erfindungsgemäßen Schaftelement verwendeten Elemente sollen möglichst klein, reibungs- und verschleißfrei ausgeführt werden. Zu große Gelenke würden den Bauraum für andere Leitungen einschränken. Verschiedene Möglichkeiten zur Realisierung der Gelenke C, C', C", D, D' sind beispielhaft in den Figuren 6a bis 6c und 7a bis 7d dargestellt. Dabei sind jeweils die Gelenke D, D' als Verbindung zwischen starren Stäben 7, 7' dargestellt, die Abschnitte der Verbindungselemente sind; in ähnlicher Weise können die Gelenke C, C', C" ausgebildet sein, die direkt am zweiten Schaftsegment B bzw. an einer proximalen Endfläche 4 des zweiten Schaftsegments B ansetzen können.

Ein sehr einfacher Aufbau eines Gelenks kann gemäß Fig. 6a durch ein Federelement in Form einer Schraubenfeder 10 realisiert werden; ebenso können beispielsweise Tellerfedern verwendet werden. Die Federn können beispielsweise aus Polymer- oder aus metallischen Werkstoffen hergestellt sein. Ein ebenfalls besonders einfacher Aufbau ergibt sich durch einen Faltenbalg 11, in dessen endseitige Öffnungen 12, 12' die Enden der Stäbe 7, 7'fest eingesetzt werden (Fig. 6b). Gemäß Fig. 6c können die Gelenke auch als Miniaturwellengelenk bzw. Kardangelenk mit zwei aufeinander senkrecht stehenden Achsen, die durch drehbar gelagerte Stifte 13, 13' realisiert sein können, ausgebildet sein. Die Gelenke können auch durch Festkörpergelenke realisiert werden.

Eine weitere Möglichkeit zur Ausbildung der Gelenke ergibt sich durch die Verwendung von Elastomerwerkstoffen. Gemäß Fig. 7a sind die Enden der Stäbe 7, 7' plattenförmig ausgebildet und mit einem Elastomerwerkstoff 15 vergossen. Gemäß Fig. 7b können die Platten 14, 14' mit Bohrungen versehen sein, um ein Verdrehen der beiden Teile gegeneinander zu vermeiden. Ein noch einfacherer Aufbau kann durch ein aufzuschrumpfendes oder aufzuklebendes Elastomerbauteil, beispielsweise einen Schlauch 16, erreicht werden, der gemäß Fig. 7c die beiden Stäbe 7, 7' miteinander verbindet. Ebenso können die Stäbe 7, 7' über ein elastisches Zwischenstück 17 miteinander verbunden sein (Fig. 7d).

Der Übersichtlichkeit halber sind nicht in allen Figuren alle Bezugszeichen angegeben. Gleiche Bezugszeichen haben in den Figuren die gleiche Bedeutung.

### Bezugszeichenliste

- A: Erstes Schaftsegment
- B: Zweites Schaftsegment
- C, C', C": Gelenk
- D, D': Gelenk
- 1: Erstes Verbindungselement
- 2: Zweites Verbindungselement
- 2': Schubstange
- 3: Drittes Verbindungselement
- 3': Schubstange
- 4: Endfläche
- 5, 5': Lumen
- 6, 6', 6", 6'": Stab
- 7, 7': Stab
- 8, 8': Längsachse
- 9: Ausleger
- 10: Schraubenfeder
- 11: Balg
- 12, 12': Öffnung
- 13, 13': Stift
- 14, 14': Platte
- 15: Elastomerwerkstoff
- 16: Schlauch
- 17: Zwischenstück
- 18: Vorsprung

## Patentansprüche

1. Schaftelement für ein endoskopisches Instrument umfassend ein erstes, proximales Schaftsegment (A) und ein zweites, distales Schaftsegment (B), wobei das zweite Schaftsegment (B) über drei Verbindungselemente (1, 2, 3) mit dem ersten Schaftsegment (A) verbunden ist, wobei das erste Verbindungselement (1) derart mit dem ersten Schaftsegment (A) verbunden ist, dass es gegenüber diesem in einer Längsrichtung nicht verschiebbar ist, und ein zweites und ein drittes Verbindungselement (2, 3) jeweils gegenüber dem ersten Schaftsegment (A) unabhängig voneinander in einer Längsrichtung verschiebbar sind **dadurch gekennzeichnet, dass** die Verbindungselemente (1, 2, 3) jeweils ein erstes zweiachsiges Gelenk (C, C', C") aufweisen, wobei die ersten Gelenke (C, C', C") ein Dreieck bilden.

2. Schaftelement nach Anspruch 1, wobei die ersten Gelenke (C, C', C") ein gleichseitiges Dreieck bilden, das in einer proximalen Endebene (4) des zweiten Segments (B) angeordnet ist.

3. Schaftelement nach Anspruch 1 oder 2, wobei das zweite und das dritte Verbindungselement (2, 3) jeweils ein zweites zweiachsiges Gelenk (D, D') aufweisen.

4. Schaftelement nach einem der vorhergehenden Ansprüche, wobei das zweite und das dritte Verbindungselement (2, 3) jeweils als zumindest abschnittweise starre Schubstange (2', 3') ausgebildet sind.

5. Schaftelement nach einem der vorhergehenden Ansprüche, wobei das zweite und das dritte Verbindungselement (2, 3) im Randbereich des ersten Segments (A) ansetzen.

6. Schaftelement nach einem der vorhergehenden Ansprüche, wobei das erste Verbindungselement (1) im Randbereich des zweiten Segments (B) ansetzt.

7. Schaftelement nach einem der Ansprüche 2 bis 5, wobei das erste Verbindungselement (1) mittig am zweiten Segment (B) ansetzt.

8. Schaftelement nach einem der vorhergehenden Ansprüche, wobei eines oder mehrere der zweiachsigen Gelenke (C, C', C", D, D') als Federgelenk, Elastomergelenk, Festkörpergelenk, Faltenbalggelenk oder als Kardangelenk ausgebildet ist bzw. sind.

9. Schaftelement nach einem der vorhergehenden Ansprüche, wobei das das erste und das zweite Verbindungselement (2, 3) durch Aktuatoren verschiebbar sind, die dem ersten Segment (A) zugeordnet sind.

10. Endoskopisches Instrument mit einem Schaft und einem Bedienteil, wobei der Schaft ein Schaftelement nach einem der vorhergehenden Ansprüche umfasst.

11. Endoskopisches Instrument nach Anspruch 10, wobei der Schaft eine Mehrzahl hintereinander angeordneter Schaftelemente nach einem der Ansprüche 2 bis 9 umfasst.

## Claims

1. Shank element for an endoscopic instrument, comprising a first, proximal shank segment (A) and a second, distal shank segment (B), wherein the second shank segment (B) is connected to the first shank segment (A) by three connecting elements (1, 2, 3), wherein the first connecting element (1) is connected to the first shank segment (A) in such a way that it is not displaceable with respect to the latter in a longitudinal direction, and a second connecting element (2) and third connecting element (3) are each displaceable, independently of each other, in a longitudinal direction with respect to the first shank segment (A), **characterized in that** the connecting elements (1, 2, 3) each have a first two-axis joint (C, C', C"), wherein the first joints (C, C', C") form a triangle.

2. Shank element according to Claim 1, wherein the first joints (C, C', C") form an equilateral triangle, which is arranged in a proximal end plane (4) of the second segment (B).

3. Shank element according to Claim 1 or 2, wherein the second connecting element (2) and third connecting element (3) each have a second two-axis joint (D, D').

4. Shank element according to one of the preceding claims, wherein the second connecting element (2) and third connecting element (3) are each designed as at least partly rigid push rods (2', 3').

5. Shank element according to one of the preceding claims, wherein the second connecting element (2) and third connecting element (3) start in the edge area of the first segment (A).

6. Shank element according to one of the preceding claims, wherein the first connecting element (1) starts in the edge area of the second segment (B).

7. Shank element according to one of Claims 2 to 5, wherein the first connecting element (1) starts centrally on the second segment (B).

8. Shank element according to one of the preceding claims, wherein one or more of the two-axis joints (C, C', C", D, D') is/are designed as a spring joint, elastomeric joint, solid-state joint, bellows joint or a cardan joint.

9. Shank element according to one of the preceding claims, wherein the second and third connecting elements (2, 3) are displaceable by actuators that are assigned to the first segment (A).

10. Endoscopic instrument with a shank and a control part, wherein the shank comprises a shank element according to one of the preceding claims.

11. Endoscopic instrument according to Claim 10, wherein the shank comprises a plurality of shank elements, according to one of Claims 2 to 9, arranged one after another.

## Revendications

1. Élément d'arbre pour un instrument endoscopique, comprenant un premier segment d'arbre proximal (A) et un deuxième segment d'arbre distal (B), le deuxième segment d'arbre (B) étant connecté au premier segment d'arbre (A) par le biais de trois éléments de connexion (1, 2, 3), le premier élément de connexion (1) étant connecté au premier segment d'arbre (A) de telle sorte qu'il ne puisse pas être déplacé par rapport à celui-ci dans une direction longitudinale, et un deuxième et un troisième élément de connexion (2, 3) pouvant être chacun déplacés indépendamment l'un de l'autre dans une direction longitudinale par rapport au premier segment d'arbre (A), **caractérisé en ce que** les éléments de connexion (1, 2, 3) présentent à chaque fois une première articulation à deux axes (C, C', C"), les premières articulations (C, C', C") formant un triangle.

2. Élément d'arbre selon la revendication 1, dans lequel les premières articulations (C, C', C") forment un triangle équilatéral qui est disposé dans un plan d'extrémité proximal (4) du deuxième segment (B).

3. Élément d'arbre selon la revendication 1 ou 2, dans lequel le deuxième et le troisième élément de connexion (2, 3) présentent chacun une deuxième articulation à deux axes (D, D').

4. Élément d'arbre selon l'une quelconque des revendications précédentes, dans lequel le deuxième et le troisième élément de connexion (2, 3) sont à chaque fois réalisés sous forme de tige de poussée au moins en partie rigide (2', 3').

5. Élément d'arbre selon l'une quelconque des revendications précédentes, dans lequel le deuxième et le troisième élément de connexion (2, 3) s'appliquent dans la région de bord du premier segment (A).

6. Élément d'arbre selon l'une quelconque des revendications précédentes, dans lequel le premier élément de connexion (1) s'applique dans la région de bord du deuxième segment (B).

7. Élément d'arbre selon l'une quelconque des revendications 2 à 5, dans lequel le premier élément de connexion (1) s'applique centralement contre le deuxième segment (B).

8. Élément d'arbre selon l'une quelconque des revendications précédentes, dans lequel une ou plusieurs des articulations à deux axes (C, C', C", D, D') est ou sont réalisées sous forme d'articulation à ressort, d'articulation élastomère, d'articulation à corps solide, d'articulation à soufflet, ou d'articulation à cardan.

9. Élément d'arbre selon l'une quelconque des revendications précédentes, dans lequel le deuxième et le troisième élément de connexion (2, 3) sont déplaçables par des actionneurs associés au premier segment (A).

10. Instrument endoscopique comprenant un arbre et une partie de commande, dans lequel l'arbre comprend un élément d'arbre selon l'une quelconque des revendications précédentes.

11. Instrument endoscopique selon la revendication 10, dans lequel l'arbre comprend une pluralité d'éléments d'arbre disposés les uns derrière les autres selon l'une quelconque des revendications 2 à 9.
